# EUROPÄISCHE PATENTSCHRIFT

(11) **EP 1 121 348 B1**
(45) Veröffentlichungstag und Bekanntmachung des Hinweises auf die Patenterteilung: **10.03.2004**
(21) Anmeldenummer: 99970386.1
(22) Anmeldetag: 01.10.1999
(51) Int. Cl.: C07D 273/01, A01N 43/88

(54) **AZADIOXACYCLOALKENE DERIVATE, VERFAHREN ZU DEREN HERSTELLUNG SOWIE DEREN VERWENDUNG ALS FUNGIZIDE UND SCHÄDLINGSBEKÄMPFUNGSMITTEL**
AZADIOXACYCLOALKENE DERIVATIVES, METHODS FOR THE PRODUCTION THEREOF AND THEIR USE AS FUNGICIDES AND PEST CONTROL AGENTS
DERIVES D'AZADIOXACYCLOALKENE, PROCEDES PERMETTANT DE LES PREPARER ET LEUR UTILISATION COMME FONGICIDES ET COMME AGENTS DE LUTTE CONTRE LES PARASITES

(30) Priorität: 15.10.1998 DE 19847592
(43) Veröffentlichungstag der Anmeldung: 08.08.2001
(73) Patentinhaber: BASF AKTIENGESELLSCHAFT, 67056 Ludwigshafen (DE)
(72) Erfinder: GEWEHR, Markus, D-56288 Kastellaun (DE); GRAMMENOS, Wassilios, D-67063 Ludwigshafen (DE); SAUTER, Hubert, D-68305 Mannheim (DE); BAYER, Herbert, D-68159 Mannheim (DE); GYPSER, Andreas, D-68159 Mannheim (DE); MÜLLER, Bernd, D-67227 Frankenthal (DE); PTOCK, Arne, D-67065 Ludwigshafen (DE); CULLMANN, Oliver, D-68199 Mannheim (DE); GROTE, Thomas, D-67105 Schifferstadt (DE); AMMERMANN, Eberhard, D-64646 Heppenheim (DE); LORENZ, Gisela, D-67434 Neustadt (DE); STRATHMANN, Siegfried, D-67117 Limburgerhof (DE); HARRIES, Volker, D-67227 Frankenthal (DE)
(74) Vertreter: Fitzner, Ulrich
(86) Internationale Anmeldenummer: PCT/EP1999/007307
(87) Internationale Veröffentlichungsnummer: WO 2000/021942

(56) Entgegenhaltungen:
- WO-A-95/04728
- WO-A-95/21153
- WO-A-95/21154
- WO-A-97/00866
- WO-A-97/15552
- WO-A-98/38857

## Beschreibung

Die vorliegende Erfindung betrifft Azadioxacycloalkene der Formel I: 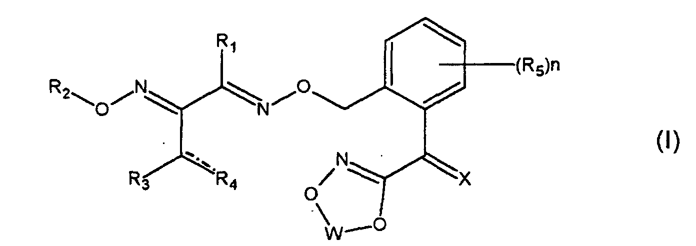 in der die Substituenten R1 bis R5 und X, der Index n und das Brückenglied W die folgende Bedeutung haben:
- R¹: Methyl, Ethyl oder Chlor;
- R²: Methyl, Ethyl, n-Propyl, i-Propyl, n-Butyl, s-Butyl, i-Butyl, Allyl oder Propargyl;
- R³: Wasserstoff oder Methyl;
- R⁴: =CR^{a}R^{b}, -CR^{d}=CR^{a}CR^{b} oder =N-OR^{c}, wobei
R^{a}, R^{b}, R^{d} unabhängig voneinander für Wasserstoff, C₁-C₄-Alkyl oder Phenyl stehen, wobei der Phenylring 1 bis 3 Substituenten ausgewählt aus der Gruppe: Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-alkylamino und C₁-C₄-Alkylthio tragen kann und
R^{c} einer der bei R² genannten Reste;
- R⁵: 6-Methyl oder 6-Chlor;
- n: 0 oder 1;
- X: CH₃-O-N=, CH₃-O-CH= oder CH₃-CH=
und
- W: unsubstituiertes Ethylen ist.

Weiterhin betrifft die Erfindung Verfahren zur Herstellung der Verbindungen I, sowie Mittel und die Verwendung der Verbindungen I zur Bekämpfung von Schadpilzen und tierischen Schädlingen.
Azadioxacycloalkene mit orthoständiger (Het)aryloxy-Gruppe sind aus den Dokumenten WO 95/04728 und WO 97/27189 bekannt.
a-Phenylacrylsäure- und a-Phenyl-a-methoximinoessigsäurederivate mit orthoständiger Bisoximether-Gruppierung sind in WO 95/21153, WO 95121154, WO 95/18789 und WO 98/38857 und ebensolche mit Trisoximether-Gruppierung in WO 97/15552 beschrieben.
Aus WO 97/00866 schließlich sind Azadioxacycloalkene mit orthoständiger α-Bisoximether-Gruppierung bekannt. Im Gegensatz zu den aus WO 97/00866 bekannten Verbindungen weisen die erfindungsgemäßen Azadioxacycloalkene am terminalen Oxim-Kohlenstoffatom der α-Bisoximether-Gruppierung einen Alkenyl- bzw. einen Alkoxyimino-alkyl-Substituenten auf.
Die in den vorstehend genannten Schriften beschriebenen Verbindungen sind als Pflanzenschutzmittel gegen Schadpilze und z.T. gegen tierische Schädlinge geeignet.
Ihre Wirkung ist jedoch in vielen Fällen nicht zufriedenstellend. Daher lag als Aufgabe zugrunde, Verbindungen mit verbesserter Wirksamkeit zu finden.

Demgemäß wurden die eingangs erwähnten Azadioxacycloalkene der Formel I gefunden. Weiterhin wurden Verfahren zur Herstellung der Verbindungen I, sowie die Verwendung der Verbindungen I und diese enthaltene Mittel zur Bekämpfung von Schadpilzen und tierischen Schädlingen gefunden. Die fungizide Wirkung ist bevorzugt.

Die Verbindungen I können auf verschiedenen Wegen erhalten werden, wobei es für die Synthese unerheblich ist, ob zunächst a) der Azadioxacycloalken-Ring (s. Schema 1) oder b) die orthoständige α-Bisoximether-Gruppierung aufgebaut wird.

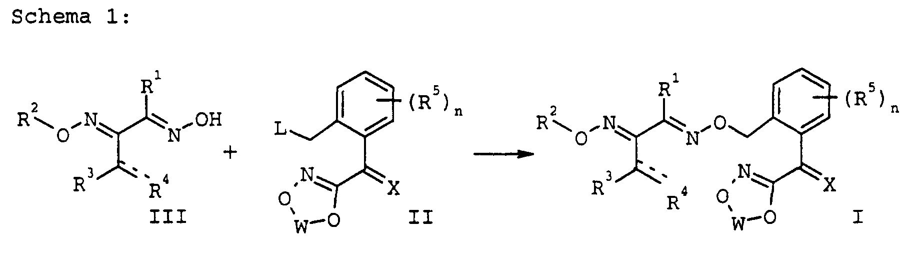

a) Insbesondere erhält man Verbindungen der Formel I dadurch, daß man eine Benzylverbindung der Formel II, in der die Reste R⁵, X und W die in Anspruch 1 gegebene Bedeutung besitzen und L für eine nucleophil austauschbare Gruppe wie Halogenid, C₁-C₄-Alkylsulfonat, C₁-C₁₂-Alkylphenylsulfonat oder Mono-C₁-C₄-alkylsulfat steht, mit einem α-Bisoxim der Formel III, in der die Substituenten R¹ bis R⁴ die in Anspruch 1 genannte Bedeutung besitzen, gegebenenfalls in Gegenwart einer Base umsetzt (s. Schema 1).

Die Benzylverbindungen II können nach literaturbekannten Synthesen hergestellt werden [vgl. WO 95/04728 und WO 97/00866]. In Formel II steht L für eine nucleophil austauschbare Gruppe, beispielsweise Halogenid, wie Fluorid, Chlorid, Bromid oder Jodid, insbesondere Chlorid oder Bromid, C₁-C₄-Alkylsulfonat wie beispielsweise Mesylat, C₁-C₁₂-Alkylphenylsulfonat wie beispielsweise Tosylat oder Mono-C₁-C₄-alkylsulfat wie beispielsweise Methylsulfat.

Die Oxime der Formel III sind ebenfalls aus der Literatur bekannt [vgl. WO 97/15552] oder können nach literaturbekannten Methoden [vgl. WO 95/21153], hergestellt werden.

Die Umsetzung der Benzylverbindung II mit dem α-Bisoxim III erfolgt in an sich bekannter Weise bei Temperaturen von -10°C bis 100°C, vorzugsweise 10°C bis 85°C, in einem inerten organischen Lösungsmittel in Gegenwart einer Base [vgl. WO 97/02255].

Geeignete Lösungsmittel sind Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid, Dimethylacetamid und besonders bevorzugt Tetrahydrofuran, Acetonitril oder Dimethylformamid. Es können auch Gemische der genannten Lösungsmittel verwendet werden.

Als Basen kommen allgemein anorganische Verbindungen, wie Alkalimetall- und Erdalkalimetallhydroxide wie Lithiumhydroxid, Natriumhydroxid, Kaliumhydroxid und Calziumhydroxid, Alkalimetall- und Erdalkalimetallhydride wie Lithiumhydrid, Natriumhydrid, Kaliumhydrid und Calziumhydrid, Alkalimetall- und Erdalkalimetallcarbonate wie Lithiumcarbonat, Kaliumcarbonat und Calziumcarbonat, sowie Alkalimetallhydrogencarbonate, wie Natriumhydrogencarbonat, Alkalimetall- und Erdalkalimetallalkoholate wie Natriummethanolat, Natriumethanolat, Kaliumethanolat, Kalium-tert.-Butanolat und Dimethoxymagnesium, außerdem organische Basen, z.B. tertiäre Amine wie Trimethylamin, Triethylamin, Di-isopropylethylamin und N-Methylpiperidin, Pyridin, substituierte Pyridine, wie Collidin, Lutidin und 4-Dimethylaminopyridin, sowie bicyclische Amine in Betracht. Besonders bevorzugt werden Natriumhydrid, Kaliumcarbonat, Natriummethanolat und Kalium-tert.-Butylat.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, III in einem Überschuß bezogen auf II einzusetzen.

b) Ein weiterer vorteilhafter Weg zur Herstellung der Verbindungen I (s. Schema 2) geht von α-Bisoxim(mono)benzylethern der Formel IV, in der R¹ bis R⁵ und X die in Anspruch 1 angegebene Bedeutung besitzen und Y für Halogen, Alkylcarbonyloxy, OH, NH₂, C₁-C₄-Alkoxy, gegebenenfalls substituiertes Phenoxy (wie beispielsweise p-Nitrophenoxy oder Pentafluorphenoxy), C₁-C₄-Alkylamino, Di-C₁-C₄-Alkylamino oder einen Aktivester wie Succinimidoxy oder Isoharnstoff steht, aus. Die Verbindungen IV werden mit Hydroxylamin oder dessen Säureadditionssalz gegebenenfalls in Gegenwart einer Base oder einer wasserentziehenden Verbindung wie N,N'-Dicyclohexylcarbodiimid und einer Verbindung L¹-W-L², in der W die in Anspruch 1 genannte Bedeutung besitzt und L¹, L² jeweils für eine nucleophil austauschbare Gruppe wie Halogenid, C₁-C₄-Alkylsulfonat, C₁-C₁₂-Alkylphenylsulfonat oder Mono-C₁-C₄-alkylsulfat stehen oder L¹ und L² gemeinsam eine Brücke -O- darstellen, umgesetzt.

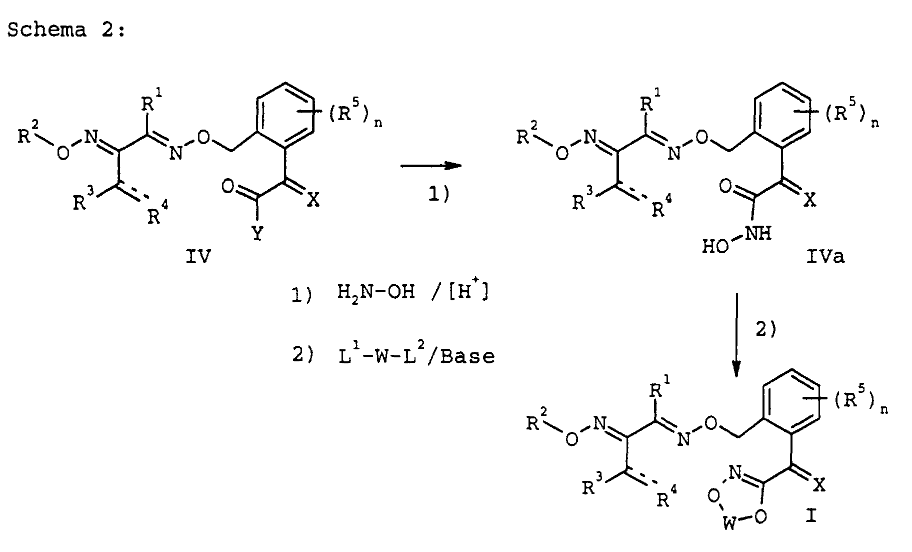

Die α-Bisoxim(mono)benzylethern der Formel IV lassen sich nach literaturbekannten Synthesen gewinnen [vgl. WO 97/15552].

Die Verbindung L¹-W-L² lassen sich, wie in WO 95/04728, WO 97/00866 und EP-A 846 691 beschrieben, darstellen.

Die in Schema 2 aufgeführten Verfahrensschritte 1) und 2) können zweistufig oder vorzugsweise einstufig, d.h. ohne Isolierung der im ersten Verfahrensschritt gebildeten Hydroxamsäure IVa ausgeführt werden. Die Durchführung kann analog den in WO 95/04728, WO 97/00866 und EP-A 846 691 beschriebenen Methoden ausgeführt werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischenund Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Verbindungen I können bei der Herstellung aufgrund ihrer C=Cund C=N-Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

In Bezug auf die -N=CR¹-C(CR³R⁴)=NOR² Doppelbindungen werden im allgemeinen hinsichtlich ihrer Wirksamkeit die E,E-Isomere der Verbindungen I bevorzugt (Konfiguration bezogen auf den Rest -CH₂O- im Verhältnis zur -C(CR³R⁴)=NOR²-Gruppe, bzw. bezogen auf den Rest -OR² im Verhältnis zur -C(R¹)-N=OCH₂-Gruppe).

Bei den in den vorstehenden Formeln angegebenen Definitionen der Symbole wurden Sammelbegriffe verwendet, die allgemein repräsentativ für die folgenden Substituenten stehen:
- Halogen: Fluor, Chlor, Brom und Jod;
- C₁-C₄-Alkyl sowie die Akylteile von C₁-C₄-Alkoxy, C₁-C₄-Alkylthio, C₁-C₄-Alkylamino, Di-C₁-C₄-alkylamin oder C₁-C₄-Alkylcarbonyl -oxy: gesättigte, geradkettige oder verzweigte Kohlenwasserstoffreste mit 1 bis 4 Kohlenstoffatomen, im einzelnen Methyl, Ethyl, Propyl, 1-Methylethyl, Butyl, 1-Methyl-propyl, 2-Methyl-propyl, 1,1-Dimethylethyl;
- C₁-C₄-Halogenalkyl sowie die Halogenalkylteile von C₁-C₄-Halogen -alkoxy: geradkettige oder verzweigte Alkylgruppen mit 1 bis 4 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;

Der Phenylrest kann einen bis drei der folgenden Reste tragen :

Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-alkylamino und C₁-C₄-Alkylthio.

Insbesondere bedeutet "subst. Phenyl" durch I bis 3 Halogenatome (vorzugsweise Fluor oder Chlor) substituiertes Phenyl.

Im Hinblick auf ihre bestimmungsgemäße Verwendung sind Azadioxacycloalkene der Formel I mit folgenden Substituenten bevorzugt, wobei die Bevorzugung jeweils für sich allein oder in Kombination zu sehen ist:

Bevorzugt sind Verbindungen der Formel I, in denen X CH₃O-CH= oder CH₃-CH= bedeutet.

Insbesondere bevorzugt sind Verbindungen der Formel I, in denen X CH₃O-N= ist.

Ebenfalls bevorzugt sind Verbindungen der Formel I, in denen n = 0 ist.

Insbesondere werden Verbindungen I bevorzugt, in denen R² für Methyl oder Ethyl steht.

Ebenfalls bevorzugt sind Verbindungen I, in denen R⁴ für =CR^{a}R^{b} steht, wobei R^{a}, R^{b} unabhängig voneinander Wasserstoff, Methyl oder Ethyl bedeuten. Insbesondere sind diese Verbindungen in Kombination mit der Bedeutung R³ Wasserstoff bevorzugt.

Insbesondere bevorzugt sind Verbindungen I, in denen R⁴ für =N-OR^{C} steht, wobei R^{c} Methyl oder Ethyl bedeutet. Insbesondere sind diese Verbindungen in Kombinatioen mit der Bedeutung R³ Methyl bevorzugt.

Außerdem bevorzugt sind Verbindungen I, in denen R⁴ für -CR^{d}=CR^{a}R^{b} steht, wobei R^{a}, R^{b} und R^{d} unabhängig voneinander Wasserstoff, Methyl oder Ethyl bedeuten. Insbesondere sind diese Verbindungen in Kombination mit der Bedeutung R³ Wasserstoff bevorzugt.

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceten, Phycomyceten* und *Basidiomyceten,* aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- *Alternaria*-Arten an Gemüse und Obst,
- *Botrytis cinerea* (Grauschimmel) an Erdbeeren, Gemüse, Zierpflanzen und Reben,
- *Cercospora arachidicola* an Erdnüssen,
- *Erysiphe cichoracearum* und *Sphaerotheca fuliginea* an Kürbisgewächsen,
- *Erysiphe graminis* (echter Mehltau) an Getreide,
- *Fusarium-* und *Verticillium-*Arten an verschiedenen Pflanzen,
- *Helminthosporium-*Arten an Getreide,
- *Mycosphaerella-*Arten an Bananen und Erdnüssen,
- *Phytophthora infestans* an Kartoffeln und Tomaten,
- *Plasmopara viticola* an Reben,
- *Podosphaera leucotricha* an Äpfeln,
- *Pseudocercosporella herpotrichoides* an Weizen und Gerste,
- *Pseudoperonospora-*Arten an Hopfen und Gurken,
- *Puccinia-*Arten an Getreide,
- *Pyricularia oryzae* an Reis,
- *Rhizoctonia*-Arten an Baumwolle, Reis und Rasen,
- *Septoria nodorum* an Weizen,
- *Uncinula necator* an Reben,
- *Ustilago*-Arten an Getreide und Zuckerrohr, sowie
- *Venturia*-Arten (Schorf) an Äpfeln und Birnen.

Die Verbindungen I eignen sich außerdem zur Bekämpfung von Schadpilzen wie *Paecilomyces variotii* im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen der Formel I sind außerdem geeignet, tierische Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor zur Bekämpfung tierischer Schädlinge eingesetzt werden. Insbesondere eignen sie sich zur Bekämpfung der folgenden tierischen Schädlinge:
- Insekten aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni und Zeiraphera canadensis,
- Käfer (Coleoptera), z.B. Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Otiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus und Sitophilus granaria,
- Zweiflügler (Diptera), z.B. Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletispomonella, Tabanus bovinus, Tipula oleracea und Tipula paludosa,
- Thripse (Thysanoptera), z.B. Frankliniella fusca, Frankliniella occidentalis, Frankliniella tritici, Scirtothrips citri, Thrips oryzae, Thrips palmi und Thrips tabaci,
- Hautflügler (Hymenoptera), z.B. Athalia rosae, Atta cephalotes, Atta sexdens, Atta texana, Hoplocampa minuta, Hoplocampa testudinea, Monomorium pharaonis, Solenopsis geminata und Solenopsis invicta,
- Wanzen (Heteroptera), z.B. Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis und Thyanta perditor,
- Pflanzensauger (Homoptera), z.B. Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum und Viteus vitifolii,
- Termiten (Isoptera), z.B. Calotermes flavicollis, Leucotermes flavipes, Reticulitermes lucifugus und Termes natalensis,
- Geradflügler (Orthoptera), z.B. Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus und Tachycines asynamorus,
- Arachnoidea wie Spinnentiere (Acarina), z.B. Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius und Tetranychus urticae,
- Nematoden wie Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus und Pratylenchus goodeyi.

Die Aufwandmenge an Wirkstoff zur Bekämpfung von tierischen Schädlingen beträgt unter Freilandbedingungen 0,1 bis 2,0, vorzugsweise 0,2 bis 1,0 kg/ha.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylenbis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-ylharnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2-[6-(2-cyanophenoxy)-pyrimidin-4-yloxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid,
- sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2-,4-triazol.

### Synthesebeispiele

### Beispiel 1

### 5-Methyl-hex-4-en-2,3-dion-2-(O-(2-((5,6-dihydro-[1,4,2]dioxazin-3-yl)-methoxyimino-methyl)-benzyl)-oxim)-3-(O-methyl-oxim) (Verbindung I-1 oder Tabelle I)

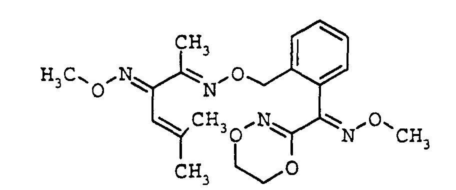

Zu einer Lösung von 1,75 g (25 mmol) Hydroxylaminhydrochlorid in 20 ml Methanol wurde eine Lösung von 3,3 g (59 mmol) Kaliumhydroxid in 20 ml Methanol gegeben. Das Gemisch wurde 5 Minuten bei Raumtemperatur gerührt, anschließend 4,5 g (12 mmol) 5-Methylhex-4-en-2,3-dion-2-(O-(2-(methoxycarbonyl-methoxyimino-methyl) -benzyl)-oxim)-3-(O-methyl-oxim) hinzugegeben und 1 h bei 40°C gerührt. Anschließend wurden 1,8 g (13 mmol) Kaliumcarbonat und 5,1 ml (59 mmol) 1,2-Dibromethan zugegeben. Der Ansatz wurde 18 h bei Raumtemperatur gerührt, im Vakuum eingeengt und der Rückstand chromatographisch gereinigt. Man erhielt 0,6 g der Titelverbindung als Öl. *270 MHz-*^{*1*}*H-NMR (CDCl*_{*3*}*),* δ *[ppm] :* 1,45 (s, 3H), 1,88 (s, 3H), 2,05 (s, 3H), 3,93 (s, 3H), 3,97 (s, 3H), 4,15 (t, 2H), 4,42 (t, 2H), 5,11 (s, 2H), 5,70 (s, 1H), 7,10-7,40 (m, 4H).

Die in den folgenden Tabellen I, II und III aufgeführten Azadioxacycloalkene lassen sich analog der im vorstehenden Synthesebeispiel wiedergegebenen Vorschrift synthetisieren.

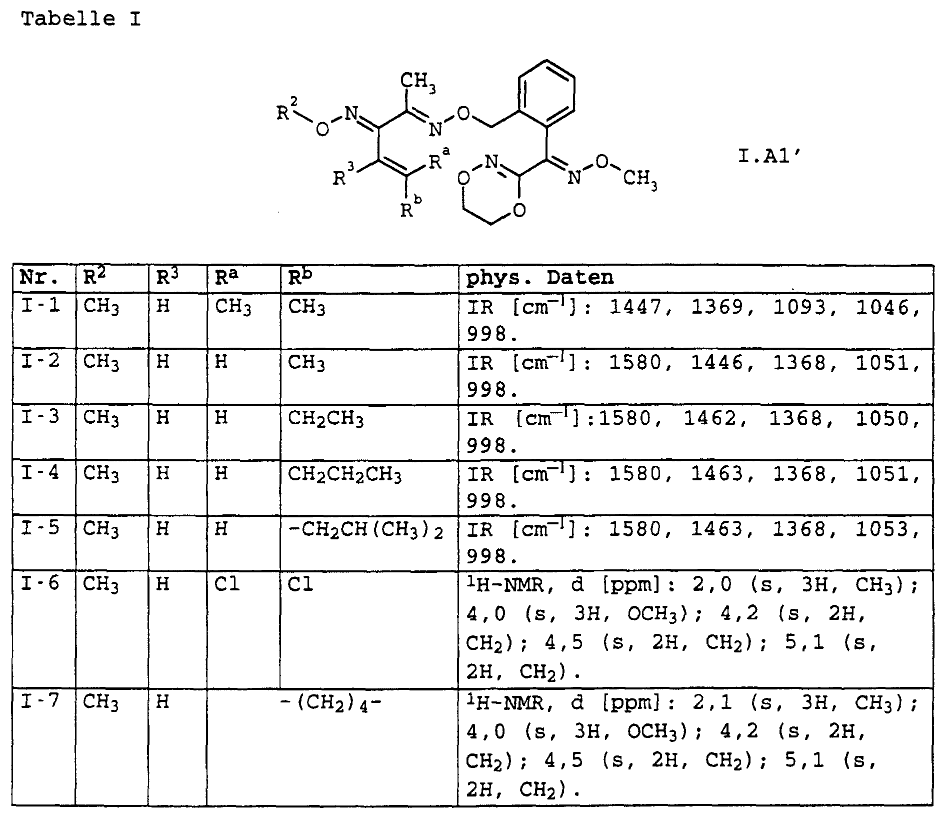

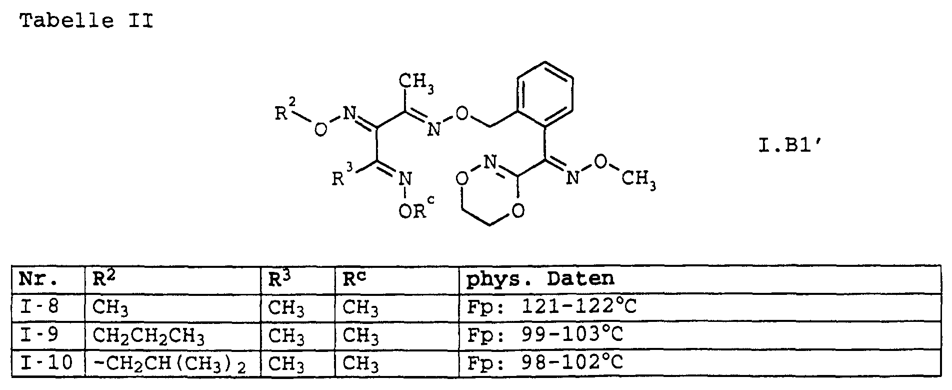

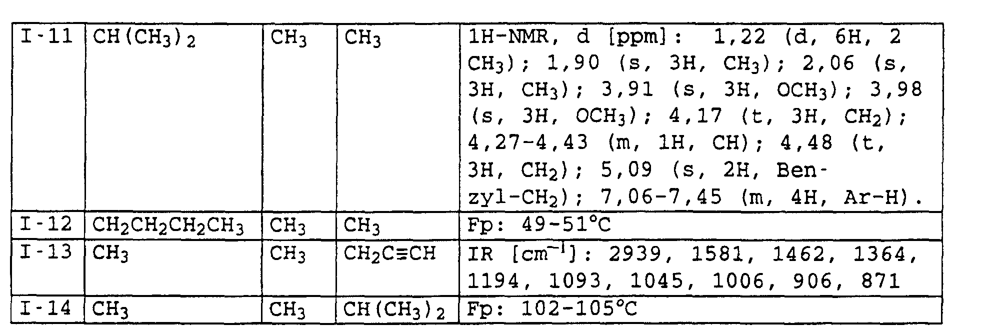

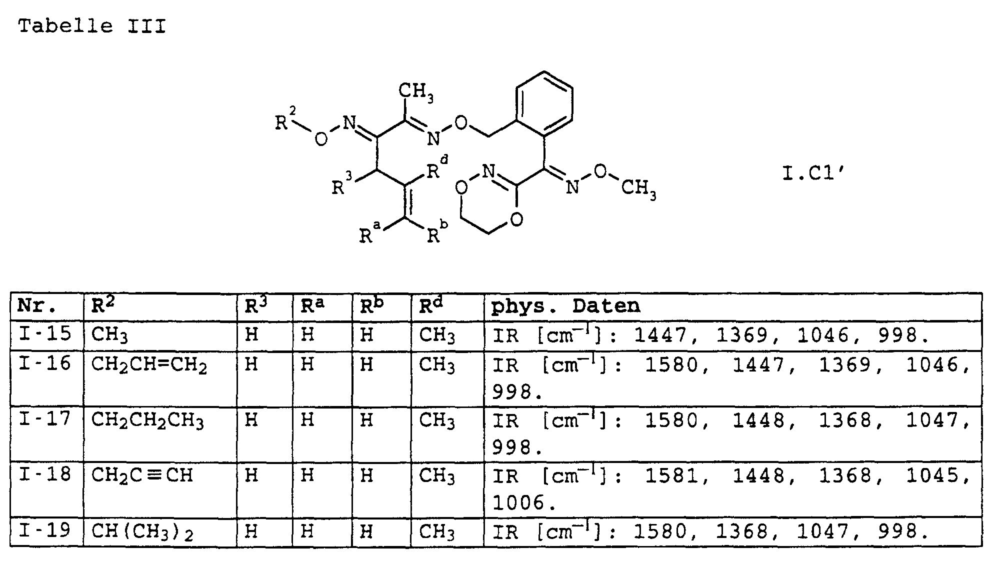

### Beispiele für die Wirkung gegen Schadpilze

Die fungizide Wirkung der Verbindungen der allgemeinen Formel I ließ sich durch die folgenden Versuche zeigen:

Die Wirkstoffe wurden getrennt oder gemeinsam als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

### Anwendungsbeispiel 1 - Wirksamkeit gegen Weizenmehltau

Blätter von in Töpfen gewachsenen Weizenkeimlingen der Sorte "Kanzler" wurden mit wäßriger Wirkstoffaufbereitung, die aus einer Stammlösung bestehend aus 10 % Wirkstoff, 63 % Cyclohexanon und 27 % Emulgiermittel angesetzt wurde, bis zur Tropfnäße besprüht und 24 Stunden nach dem Antrocknen des Spritzbelages mit Sporen des Weizenmehltaus (*Erysiphe graminis* forma specialis *tritici*) bestäubt. Die Versuchspflanzen wurden anschließend im Gewächshaus bei Temperaturen zwischen 20 und 24° C und 60 bis 90 % relativer Luftfeuchtigkeit aufgestellt. Nach 7 Tagen wurde das Ausmaß der Mehltauentwicklung visuell in % Befall der gesamten Blattfläche ermittelt.

In diesem Test zeigten die mit 16 ppm der Wirkstoffe I-1 bis I-7, I-13 und I-14 behandelten Pflanzen bis zu 10 % Befall, während unbehandelte Pflanzen zu 90 % geschädigt waren.

### Anwendungsbeispiel 2 - Wirksamkeit gegen Plasmopara viticola

Blätter von Topfreben der Sorte "Müller-Thurgau" wurden mit wäßriger Wirkstoffaufbereitung, die mit einer Stammlösung aus 10 % Wirkstoff, 63 % Cyclohexanon und 27 % Emulgiermittel angesetzt wurde, bis zur Tropfnässe besprüht. Um die Dauerwirkung der Substanzen beurteilen zu können, wurden die Pflanzen nach dem Antrocknen des Spritzbelages für 7 Tage im Gewächshaus aufgestellt. Erst dann wurden die Blätter mit einer wäßrigen Zoosporenaufschwemmung von *Plasmopara viticola* inokuliert. Danach wurden die Reben zunächst für 48 Stunden in einer wasserdampfgesättigten Kammer bei 24° C und anschließend für 5 Tage im Gewächshaus bei Temperaturen zwischen 20 und 30°C aufgestellt. Nach dieser Zeit wurden die Pflanzen zur Beschleunigung des Sporangienträgerausbruchs abermals für 16 Stunden in eine feuchte Kammer gestellt. Dann wurde das Ausmaß der Befallsentwicklung auf den Blattunterseiten visuell ermittelt.

In diesem Test zeigten die mit 63 ppm der Wirkstoffe I-1 bis I-7, I-11 bis I-14 und I-16 bis I-18 behandelten Pflanzen bis zu 15 % Befall, während unbehandelte Pflanzen zu 90 % geschädigt waren.

### Beispiele für die Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a. als 0,1%-ige Lösung in Aceton oder
b. als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl)
aufbereitet und entsprechend der gewünschten Konzentration mit Aceton im Fall von a. bzw. mit Wasser im Fall von b. verdünnt.

Nach Abschluß der Versuche wurde die jeweils niedrigste Konzentration ermittelt, bei der die Verbindungen im Vergleich zu unbehandelten Kontrollversuchen noch eine 80 bis 100%-ige Hemmung bzw. Mortalität hervorriefen (Wirkschwelle bzw. Minimalkonzentration).

## Patentansprüche

1. Azadioxacycloalkene der Formel I, 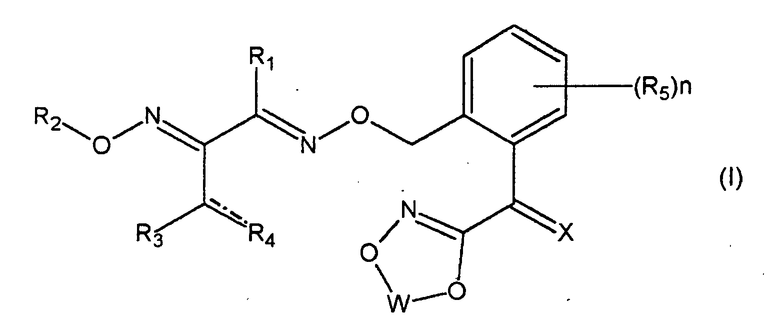 in der die Substituenfcen R1 bis R5 und X, der Index n und das Brückenglied W die folgende Bedeutung haben:
R¹ Methyl, Ethyl oder Chlor;
R² Methyl, Ethyl, n-Propyl, i-Propyl, n-Butyl, s-Butyl, i-Butyl, Allyl oder Propargyl;
R³ Wasserstoff oder Methyl;
R⁴ =CR^{a}R^{b}, -CR^{d}=CR^{a}CR^{b} oder =N-OR^{c}, wobei
R^{a}, R^{b}, R^{d} unabhängig voneinander für Wasserstoff, C₁-C₄-Alkyl oder Phenyl stehen, wobei der Phenylring 1 bis 3 Substituenten ausgewählt aus der Gruppe: Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄alkylamino und C₁-C₄-Alkylthio tragen kann und
R^{c} einer der bei R² genannten Reste;
R⁵ 6-Methyl oder 6-Chlor;
n 0 oder 1;
X CH₃-O-N=, CH₃-O-CH= oder CH₃-CH= und
W unsubstituiertes Ethylen ist.

2. Verfahren zur Herstellung der Verbindungen der Formel I nach Anspruch I, **dadurch gekennzeichnet, daß** man eine Benzyl Verbindung der Formel II, 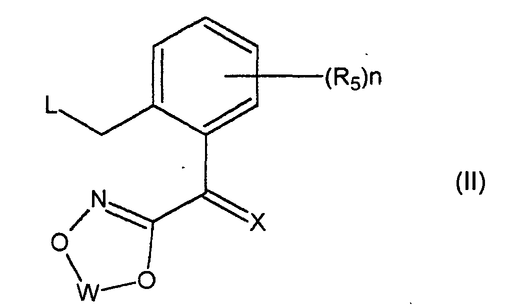 in der die Reste R⁵, X und W die in Anspruch I gegebene Bedeutung besitzen und L für eine nucleophil austauschbare Gruppe wie Halogenid, C₁-C₄-Alkylsulfonat, C₁-C₁₂-Alkylphenylsulfonat oder Mono-C₁-C₄alkylsulfat steht, mit einem α-Bisoxim der Formel III, 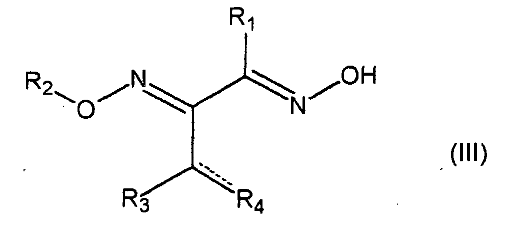 in der die Substituenten R¹ bis R⁴ die in Anspruch I genannte Bedeutung besitzen, gegebenenfalls in Gegenwart einer Base umsetzt.

3. Verfahren zur Herstellung der Verbindungen der Formel I nach Anspruch I, **dadurch gekennzeichnet, daß** man einen α-Bisoxim(mono)benzylether der Formel IV, 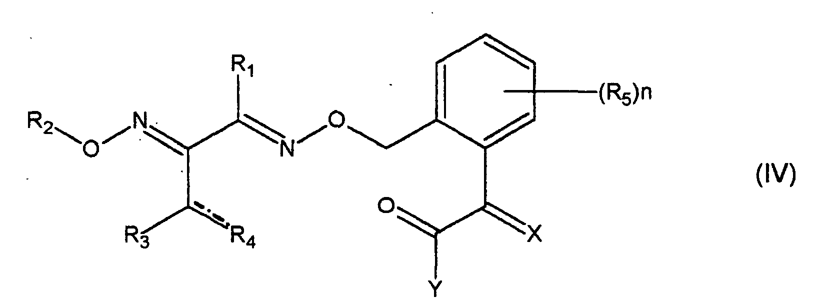 in der R¹ bis R⁵ und X die in Anspruch I angegebene Bedeutung besitzen und Y für Halogen, C₁-C₄-Alkylcarbonyloxy, OH, NH₂, C₁-C₄-Alkoxy, gegebenenfalls substituiertes Phenoxy, C₁-C₄-Alkylamino, Di-C₁-C₄-Alkylamino oder einen Aktivester steht, mit Hydroxylamin oder dessen Säureadditionssalz und einer Verbindung L¹-W-L², in der W die in Anspruch I genannte Bedeutung besitzt und L¹, L² jeweils für eine nucleophil austauschbare Gruppe wie Halogenid, C₁-C₄-Alkylsulfonat, C₁-C₁₂-Alkylphenylsulfonat oder Mono-C₁-C₄-alkylsulfat stehen oder L¹ und L² gemeinsam eine Brücke -0- darstellen, gegebenenfalls in Gegenwart einer Base oder eines wasserentziehenden Mittels umsetzt.

4. Zur Bekämpfung von tierischen Schädlingen oder Schadpilzen geeignetes Mittel, enthaltend einen festen oder flüssigen Träger Stoff und eine Verbindung der Formel I gemäß Anspruch I.

5. Verfahren zur Bekämpfung von Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der Formel I gemäß Anspruch I behandelt.

6. Verfahren zur Bekämpfung von tierischen Schädlingen, **dadurch gekennzeichnet, daß** man die tierischen Schädlinge oder die vor ihnen zu schützenden Materialien, Pflanzen, den Boden oder Saatgüter mit einer wirksamen Menge einer Verbindung der Formel I gemäß Anspruch I behandelt.

## Claims

1. An azadioxacycloalkene of the formula I, 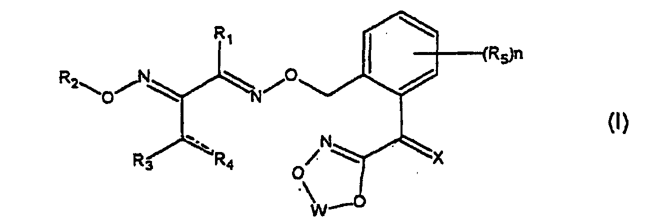 in which the substituents R1 to R5 and X, the index n and the bridge member W have the following meanings:
R¹ is methyl, ethyl or chlorine;
R² is methyl, ethyl, n-propyl, isopropyl, n-butyl, s-butyl, isobutyl, allyl or propargyl;
R³ is hydrogen or methyl;
R⁴ is =CR^{a}R^{b}, CR^{d}=CR^{a}R^{b} or =N-OR^{c}, where
R^{a}, R^{b}, R^{d} independently of one another are each hydrogen, C₁-C₄-alkyl or phenyl, where the phenyl ring may carry from 1 to 3 substituents selected from the group: halogen, cyano, nitro, C₁-C₄-alkyl, C₁-C₄-haloalkyl, C₁-C₄-alkoxy, C₁-C₄-haloalkoxy, C₁-C₄-alkylamino, di-C₁-C₄-alkylamino and C₁-C₄-alkylthio and
R^{c} is one of the radicals mentioned under R²;
R⁵ is 6-methyl or 6-chlorine;
n is 0 or 1;
X is CH₃-O-N=, CH₃-O-CH= or CH₃-CH= and
W is unsubstituted ethylene.

2. A process for preparing compounds of the formula I as claimed in claim 1, which comprises reacting a benzvl compound of the formula II 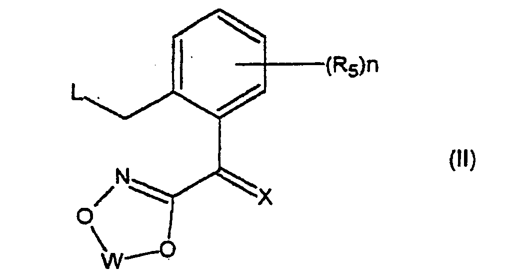 in which the radicals R⁵, X and W are each as defined in claim 1 and L is a nucleophilically replaceable group, such as halide, C₁-C₄-alkylsulfonate, C₁-C₁₂-alkylphenylsulfonate or mono-C₁-C₄-alkyl sulfate, with an α-bisoxime of the formula III. 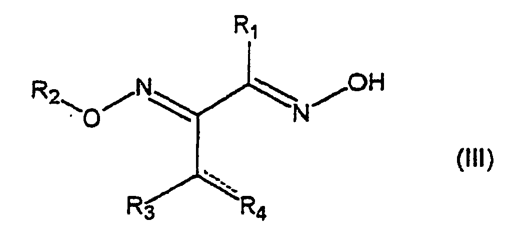 in which the substituents R¹ to R⁴ are each as defined in claim 1, if appropriate in the presence of a base.

3. A process for preparing the compounds of the formula I as claimed in claim 1, which comprises reacting an α-bisoxime (mono)benzyl ether of the formula IV, 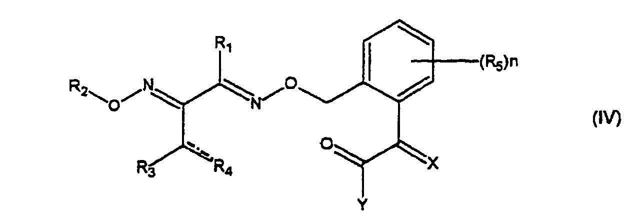 in which R¹ to R⁵ and X are each as defined in claim 1 and Y is halogen, C₁-C₄-alkylcarbonyloxy, OH, NH₂, C₁-C₄-alkoxy, unsubstituted or substituted phenoxy, C₁-C₄-alkylamino, di-C₁-C₄-alkylamino or an active ester with hydroxylamine or its acid addition salt and a compound L¹-W-L² in which W is as defined in claim 1 and L¹ and L² are each a nucleophilically replaceable group, such as halide, C₁-C₄-alkylsulfonate, C₁-C₁₂-alkylphenylsulfonate or mono-C₁-C₄-alkyl sulfate, or L¹ and L² together are a bridge -O-, if appropriate in the presence of a base or a dehydrating agent.

4. A composition which is suitable for controlling animal pests or harmful fungi, comprising a solid or liquid carrier and a compound of the formula I as claimed in claim 1.

5. A method for controlling harmful fungi, wherein the fungi or the materials, plants, the soil or the seed to be protected against fungal attack are treated with an effective amount of a compound of the formula I as claimed in claim 1.

6. A method for controlling animal pests, wherein the animal pests or the materials, plants, the soil or the seed to be protected from them are treated with an effective amount of a compound of the formula I as claimed in claim 1.

## Revendications

1. Azadioxacycloalcènes de formule I : 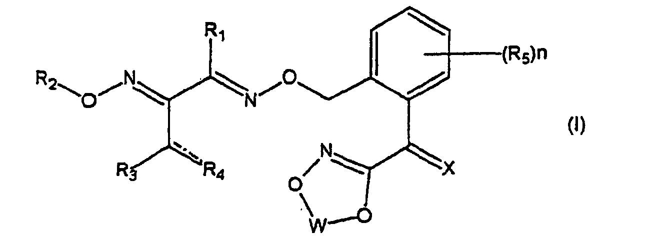 dans laquelle les substituants R¹ à R⁵ et X, l'indice n et le chaînon pontant W ont les significations suivantes :
R¹ représente le groupe méthyle, éthyle ou un atome de chlore ;
R² représente le groupe méthyle, éthyle, n-propyle, isopropyle, n-butyle, sec-butyle, isobutyle, allyle ou propargyle ;
R³ représente un atome d'hydrogène ou le groupe méthyle ;
R⁴ représente =CR^{a}R^{b}, -CR^{d}=CR^{a}CR^{b} ou =N-OR^{c},
R^{a}, R^{b}, R^{d} représentant, indépendamment les uns des autres, un atome d'hydrogène, un groupe alkyle en C₁-C₄ ou phényle, le noyau phényle pouvant porter de 1 à 3 substituants choisis dans l'ensemble constitué par : des atomes d'halogène, des groupes cyano, nitro, alkyle en C₁-C₄, halogénoalkyle en C₁-C₄, alcoxy en C₁-C₄, halogénoalcoxy en C₁-C₄, alkyl(C₁-C₄)amino, dialkyl(C₁-C₄)amino et alkyl-(C₁-C₄)thio et
R^{c} étant l'un des radicaux cités pour R² ;
R⁵ représente le groupe 6-méthyle ou 6-chloro ;
n est 0 ou 1 ;
X est CH₃-O-N=, CH₃-O-CH= ou CH₃-CH= et
W est un groupe éthylène non substitué.

2. Procédé pour la préparation des composés de formule I selon la revendication 1, **caractérisé en ce qu'**on fait réagir un composé benzyle de formule II, 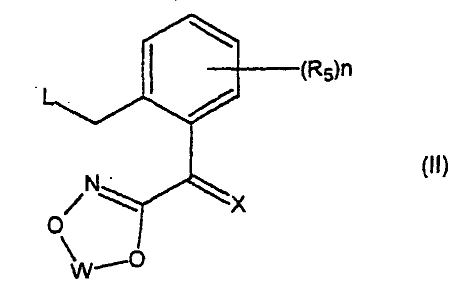 dans lequel les radicaux R⁵, X et W ont les significations données dans la revendication 1 et L représente un groupe séparable par substitution nucléophile, tel qu'un halogénure, un groupe alkyl(C₁-C₄)-sulfonate, alkyl(C₁-C₁₂)phénylsulfonate ou monoalkyl(C₁-C₄) sulfate, avec une α-bisoxime de formule III, 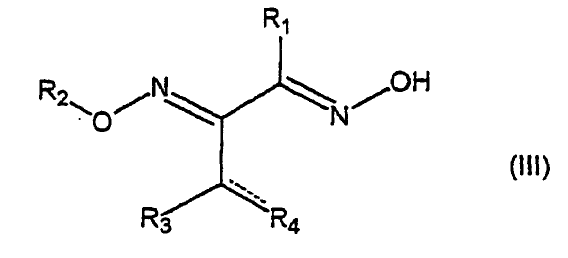 dans laquelle les substituants R¹ à R⁴ ont les significations données dans la revendication 1, éventuellement en présence d'une base.

3. Procédé pour la préparation des composés de formule I selon la revendication 1, **caractérisé en ce qu'**on fait réagir un α-bisoxime(mono)benzyléther de formule IV, 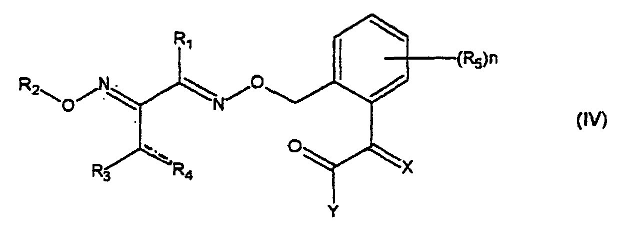 dans laquelle R¹ à R⁵ et X ont les significations données dans la revendication 1 et Y représente un atome d'halogène, un groupe alkyl(C₁-C₄)carbonyloxy, OH, NH₂, un groupe alcoxy en C₁-C₄, un groupe phénoxy éventuellement substitué, alkyl(C₁-C₄)-amino, dialkyl-(C₁-C₄)-amino ou un ester actif, avec de l'hydroxylamine ou un sel d'addition avec un acide de celle-ci, et un composé L¹-W-L², dans lequel W a la signification donnée dans la revendication 1 et L¹, L² représentent chacun un groupe séparable par substitution nucléophile, tel qu'un halogénure, un groupe alkyl(C₁-C₄)-sulfonate, alkyl(C₁-C₁₂)phénylsulfonate ou monoalkyl(C₁-C₄)-sulfate, ou L¹ et L² représentent ensemble un pont -O-, éventuellement en présence d'une base ou d'un agent déshydratant.

4. Composition appropriée à la lutte contre des champignons nuisibles ou des ravageurs animaux, contenant une substance de support solide ou liquide et un composé de formule I selon la revendication 1.

5. Procédé pour la lutte contre des champignons nuisibles, **caractérisé en ce qu'**on traite les champignons, ou les matériaux, les plantes, le sol ou les semences à protéger contre l'attaque de champignons, par une quantité efficace d'un composé de formule I selon la revendication 1.

6. Procédé pour la lutte contre des ravageurs animaux, **caractérisé en ce qu'**on traite les ravageurs animaux ou les matériaux, plantes, le sol ou les semences à protéger contre ceux-ci, par une quantité efficace d'un composé de formule I selon la revendication 1.
